# EUROPÄISCHE PATENTSCHRIFT

(11) **EP 0 325 186 B1**
(45) Veröffentlichungstag und Bekanntmachung des Hinweises auf die Patenterteilung: **05.10.1994**
(21) Anmeldenummer: 89100653.8
(22) Anmeldetag: 16.01.1989
(51) Int. Cl.: A61M 5/00, A61M 5/14, F16K 7/06

(54) **Einrichtung zur Absperrung von Kunststoffschläuchen, insbesondere Infusionsschläuchen,für Luftfallen**
Shut-off device for closing synthetic tubes, especially infusion tubes, for air traps
Organe d'arrêt pour fermer des tubes synthétiques, en particulier des tubes d'infusion, pour des pièges d'air

(30) Priorität: 21.01.1988 DE 3801577
(43) Veröffentlichungstag der Anmeldung: 26.07.1989
(73) Patentinhaber: Hübner, Karl-Alexander, D-76228 Karlsruhe (DE)
(72) Erfinder: Hübner, Karl-Alexander, D-76228 Karlsruhe (DE)
(74) Vertreter: Hubbuch, Helmut, Dipl.-Ing

(56) Entgegenhaltungen:
- EP-A- 0 150 666
- DE-A- 3 435 781
- DE-C- 3 627 011

## Beschreibung

Die Erfindung besteht aus von einer Luftfalle zur Absperrung von Kunsstoffschläuchen, insbesondre Infusionschläuchen mit den im Oberbegriff des Anspruchs 1 angegebenen Merkmalen.

Es sind Luftfallen für diese Zwecke mit Schlauchabklemmung vermittels gegen Federwirkung kolbengeführten Druckbolzen bekannt, welche dafür sorgen sollen, daß bei Infusionen beim Ausgehen der Infusionsflüssigkeit oder anderen Störungen, wenn diese unter Druck der Blutbahn des Patienten zugeführt wird, unterbrochen wird, sobald Luftblasen folgen, damit diese nicht in die Blutbahn gedrückt werden, was zum Tod des Patienten führen würde. So wird beispielsweise mit Ozon angereichertes Patientenblut unter Sauerstoffdruck aus der Blutinfusionsflasche in die Blutbahn des Patienten zurückgeführt.

Durch die DE-A-34 35 781 ist eine Abspereinrichtung für Kunststoffschläuche bekannt, welche in einer Druckkammer eine Membrane aufweist, die einen hin- und herbewegbaren Stößel betätigt. Unterhalb der Membrane und mit dem Stößel verbunden ist ein Kolben angeordnet, welcher in einem Zylinder hin- und herbewegbar ist. Bei einer Druckbeaufschlagung des Zylinderraums wird der Kolben gegen eine Federwirkung aus dem Zylinder herausgedrückt und der Stößel klemmt dabei einen Schlauch ab. Liegt im Zylinderraum kein Druck an, so wird der stößel mit Kolben aufgrund der Federwirkung in den Zylinderraum hinuntergedrückt, wobei der Schlauch freigegeben wird. Nachteilig bei dieser durch die DE-A-34 35 781 bekannten Luftfalle ist, daß im Falle eines Druckabfalls im Zylinderraum die Klemmwirkung aufgehoben wird.

Desweiteren ist durch die EP-A-0 150 666 ein Verschluß an einem Harnkatheder bekannt, bei dem der Verschluß unter Federwirkung normalerweise geschlossen ist. Dies wird dadurch erzielt, daß der Verschluß einen hin- und herbewegbaren Stößel aufweist, welcher durch Federwirkung einen Schlauch abklemmt. Der Stößel wird hierbei manuell betätigt. Bei manuellem Druck wird der Schlauchdurchgang freigegeben und bei manueller Druckentlastung wird er durch den Stößel unter Federwirkung abgeklemmt.

Bedingt durch die manuelle Betätigung kann durch den aus der EP-A-0 150 666 bekannten Verschluß keine selbsttätige Abschaltung erfolgen. Desweiteren kann die Funktionsfähigkeit des Verschlusses infolge von Ermüdungsbruch oder. Verschleiß der Federn gefährdet sein kann.

Der vorliegenden Erfindung liegt daher die Aufgabe zugrunde, die bekannte Einrichtung zur Absperrung von Kunststoffschläuchen so weiterzubilden, daß in Verwendung für eine Luftfalle in Notfällen selbsttätig eine sichere und zuverlässige Klemmwirkung erzielt wird .

Diese Aufgabe wird durch die Merkmale des Anspruchs 1 gelöst. Vorteilhafte Weiterbildungen sind Gegenstand der Unteransprüche.

Die erfindungsgemäße Luftfalle zur Absperrung von Kunststoffschläuchen, insbesondere Infustionsschläuchen zeichnet sich dadurch aus, daß am Stößel angreifende, jeweils mit einem Schaltstrom durchflossene Druckfedern vorgesehen sind, wobei beim Bruch wenigstens einer Feder diese den Schaltstrom unterbricht und damit den Gaseinlaß sperrt. Dies hat den Vorteil, daß bei Ermüdung oder beim Bruch einer Feder stets sichergestellt wird, daß der Gaseinlaß gesperrt wird, wodurch eine selbsttätige Abschaltung vollzogen wird.

In der Zeichnung ist beispielsweise eine bevorzugte Ausführungsform der erfindungsgemäßen Luftfalle dargestellt und nachfolgend beschrieben und zwar zeigen:
- Figur 1: einen Schnitt durch eine Luftfalle beim öffnen,
- Figur 2: einen Schnitt der Luftfalle nach Figur 1 in geschlossener Stellung und
- Figur 3: einen Schnitt durch eine Desamtanordnung.

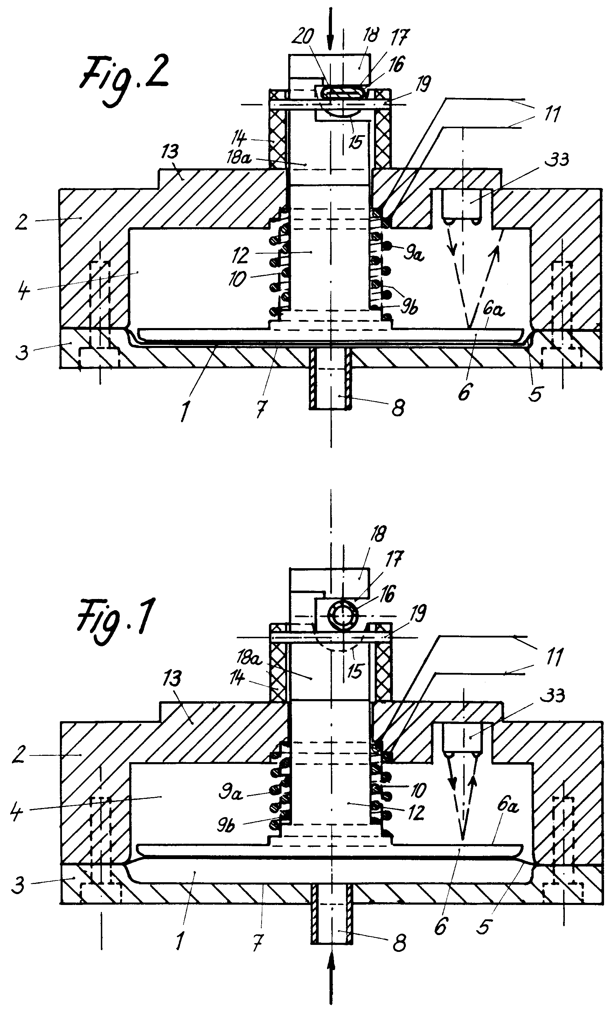

Wie aus der Zeichnung Fig. 1 und 2 ersichtlich ist, wird die Druckkammer 1 gebildet durch die beiden miteinander verbundenen Gehäuseteile 2 und 3, insbes. aus Kunststoff mit eingebrachter Ausnehmung 4, in welcher eine tellerförmige Membrane 5, z.B. als Teflonmembrane eingespannt ist. Dieser Membrane 5 liegt einerseits der Plankopf 6 an, während andererseits der Ausnehmungsboden 7 mit Druckleitungsanschluß 8 liegt.

Der Plankopf 6 sitzt auf zwei konzentrisch ineinanderliegenden Druckfedern 9a, 9b, welche vorzugsweise gegeneinander, z.,B. mittels Isolierspirale 10, isoliert und durch einen Schaltstrom 11 durchflossen sind, wodurch dieser bei Federbruch unterbrochen und damit der Druckleitungsanschluß 8 gesperrt wird, wie dies später geschildert ist.

Der Plankopf 6 reicht mit seinem Führungsstößel 12 durch eine einem Gehäusedeckel 13 aufsitzende Hülse 14, welche durch Einwölbungen 15 für einen Kunststoffschlauch 16 als Durchgang 17 dient und mittels Umgriff 18 denselben (17) verschließt, solange die Druckkammer 1 nicht unter Gasdruck steht. Die Hülse 14 ist auf dem Gehäusedeckel 13 befestigt oder auch mit diesem aus einem Stück und weist mit beidseitigen Einwölbungen 15 als Schlauchdurchgang 17 mittig einen Quersteg 19 auf, welcher zusammen mit einem Umgriff 18 und Schlitzung 18a für den Quersteg 19 eine Schlauchabklemmung 20 bewirkt.

Es ist beidseits des Schlauchdurchgangs 17 je eine Lichtschranke (nicht dargestellt) vorgesehen, welche bei im Schlauch 16 durchlaufender Flüssigkeit, wie Blut geöffnet sind und bei Durchtritt von Luftblasen schließen und dabei über eine hier nicht dargestellte Steuerung einen Stromschalter 22 öffnen.
Beidseits am Schlauchdurchgang 17 sind übliche Halterungen zur Schlauchanlage und -entnahme vorgesehen, wie dies hier nicht besondres dargestellt ist.

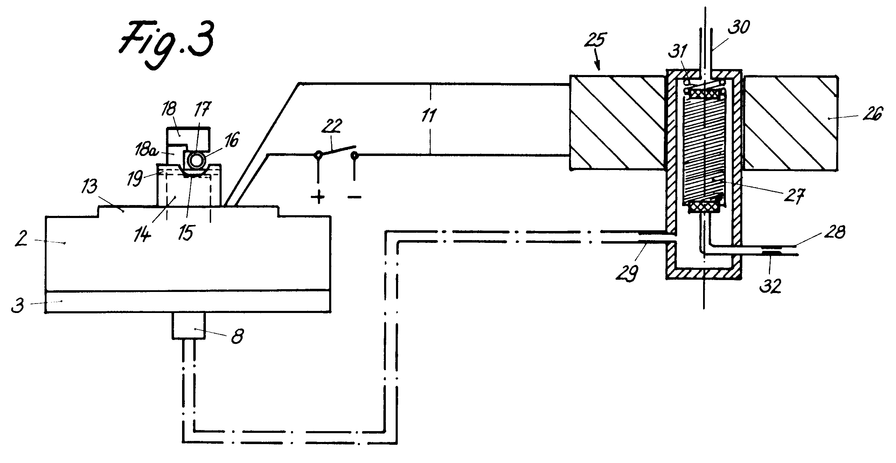

Die Öffnung der Luftfalle, d.h. des Schlauchdurchgangs 17 des Kunststoffschlauchs 16 am Umgriff 18 erfolgt gegen die Wirkung der Feder 9a, 9b bei Beaufschlagung des Plankopfes 6 mit Membran 5 durch Gaseinlaß (bei 8). Dieser wird durch ein Dreiwegeventil 25 gesteuert, bei welchem bei Bestromung einer Spule 26 ein Magnetkern 27 einen Ganseingang 28, z.B. von einer Sauerstoffdruckflasche und Durchgang 29 zum Gaseinlaß 8 für die Druckkammer 1 öffnet und bei Stromunterbrechung schließt und einen Gasausgang 30 zur Atmosphäre öffnet. Hierfür steht der Magnetkern 27 unter Wirkung einer Feder 31, so daß er bei Stromunterbrechung an der Spule 26 die Sperrung des Durchgangs (Gaseingangs) 29 unter zusätzlicher Sicherung durch sein Eigengewicht bewirkt und den Gasausgang 30 zur Atmosphäre hin öffnet, wie dies schematisch in der Zeichnung nach Figur 3 dargestellt ist. Im übrigen ist der Gaseingang 28 gegenüber dem Durchgang 29 und Gasausgang 30 bei 32 gedrosselt, damit bei gebrochener Feder 31 der Gasstrom soweit gedrosselt wird, daß der Überdruck in der Leitung durch den induzierten pneumatischen Widerstand nicht ausreicht, um die Luftfalle zu öffnen bzw. beim Abschalten des Magnetventils der vorhandene Überdruck durch den Gasausgang 30 mit dem zuströmenden Gas aus Leitung 28 gegen die Atmosphäre entlüftet werden kann. Das Eigengewicht des Magnetkerns 27 is dabei größer zu wählen als die pneumatische Anpreßkraft zum Gasausgang 30.

Eine weitere Reflektions-Lichtschranke 33 kann von der Oberfläche 6a des Plankopfes 6 gesteuert werden und zwar vergeht zwischen dem gedrosselten (32) Gaseingang 28 bei Betätigung des Mgnetkerns 27 durch Bestromung der Spule 26 und dem Füllen der Druckkammer 1 mit Betätigung des an der Membrane 5 anliegenden Plankopfes 6 gegen Wirkung der Feder 9a, 9b bis zum vollen öffnen des Schlauchdurchgangs 17 der Luftfalle mittels Umgriff 18 eine vorbestimmte Zeit. Wird nun diese vorgegebene Zeit unterschritten, so sind die Federn 9a, 9b bzw. eine hiervon entweder erlahmt oder gebrochen und die Abklemmung am Schlauchdurchgang 17 wird in diesem Falle durch eine entsprechende Zeitmeß-Vergleichssteuerung durch Entlüftung der Druckkammer 1, z.B. über Stromabschaltung an der Spule 26 bewirkt. Diese Anordnung kann zusätzlich zu der Stromdurchgangsüberwachung der Federn 9a, 9b, wie vorgeschildert, zur Funktionssicherheit der Luftfalle eingebaut werden.

Zufolge des großflächigen Plankopfs 6 arbeitet diese Luftfalle mit geringeren Drücken; im übrigen läßt sich die Gesamtanordnung bei entspr. kürzeren Druckfedern flacher bauen als in der Zeichnung dargestellt.

## Patentansprüche

1. Absperrung einrichtung für Kunststoffschläuche, insbesondere Infusionsschlauche, mit einem Gehäuse (2,3), in dem eine Druckkammer (1) vorgesehen ist, in welcher eine Membran (5) angeordnet ist u. welche über ein in einer Zuleitung angeordnetes, elektrisch betätigbares Ventil (25) mit Gas versorgt wird, mit einem hin-u.herbewegbaren Stößel (12), der einerseits einen Plankopf (6) aufweist, der auf der Membran (5) aufliegt, u. andrerseits einen Umgriff (18) aufweist, der unter Federwirkung einen Schlauchdurchgang (17) beaufschlagt, so daß bei unter Gasdruck stehender Membran (5) bzw. unter Federwirkung der Schlauchdurchgang (17) freigegeben oder der Schlauch abgeklemmt wird,
**dadurch gekennzeichnet, daß** in Verwendung der Absperreinrichtung für eine Luftfalle die am Stößel (12) angreifenden Druckfedern (9a,9b) jeweils von einem Schaltstrom durchflossen werden, wobei beim Bruch wenigstens einer Druckfeder (9a,9b) diese den Schaltstrom unterbricht u. somit der Schlauchdurchgang (17) gesperrt wird.

2. Absperreinrichtung für eine Luftfalle nach Anspruch 1, dadurch gekennzeichnet, daß das Gehäuse (2,3) zwei miteinander verbundene, jeweils eine Aussparung aufweisende Gehäuseteile (2,3) hat, wobei die Membran (5) in Tellerform von ihnen eingespannt wird u. dabei einerseits die Druckkammer (1) u. andrerseits eine den Plankopf (6) u. die Druckfedern (9a,9b), aufnehmende Aussparung (4) bildet.

3. Absperreinrichtung für eine Luftfalle nach Anspruch 1 oder 2, dadurch gekennzeichnet, daß zwei konzentrisch ineinanderliegende Druckfedern (9a,9b) am Plankopf (6) angreifen.

4. Absperreinrichtung für eine Luftfalle nach Anspruch 3, dadurch gekennzeichnet, daß die Druckfedern (9a,9b) gegeneinander isoliert sind.

5. Absperreinrichtung für eine Luftfalle nach einem der Ansprüche 1-4, dadurch gekennzeichnet, daß sich der Stößel (12) durch eine auf einem Gehäusedeckel (13) angeordnete Hülse (14), welche als Durchgang für einen Schlauch (16) dient, erstreckt.

6. Absperreinrichtung für eine Luftfalle nach Anspruch 5, dadurch gekennzeichnet, daß die Hülse (14) mit beidseitigen Einwölbungen (15) für den Durchgang des Schlauchs (16) mittig dazu senkrecht einen Quersteg (19) aufweist, welcher zusammen mit dem Umgriff (18) die Schlauchabklemmung bewirkt.

7. Absperreinrichtung für eine Luftfalle nach einem der vorhergehenden Ansprüche, dadurch gekennzeichnet, daß beidseitig des Durchgangs für den Schlauch (16) je eine Lichtschranke angeordnet ist, die jeweils bei im Schlauch (16) durchlaufender Farbflüssigkeit geöffnet ist u. bei Durchtritt von Luftblasen schließt u. zusätzlich über eine Steuerung den Gaseinlaß sperrt.

8. Absperreinrichtung für eine Luftfalle nach einem der vorhergehenden Ansprüche, dadurch gekennzeichnet, daß am Durchgang des Schlauchs (16) beidseits Halterungen zur Schlaucheinlage u. -entnahme vorgesehen sind.

9. Absperreinrichtung für eine Luftfalle nach einem der vorhergehenden Ansprüche, dadurch gekennzeichnet, daß in der Ausnehmung (4) eine Reflektionsschranke (33) sowie eine Zeitmeß-u.Vergleichssteuerung vorgesehen sind, wobei bei einer Abweichung zu. einem Sollwert u. einem Istwert zum Betätigen des an der Membran (5) anliegenden Plankopfs (6) eine Entlüftung der Druckkammer (1) bewirkt wird.

## Claims

1. Blocking device for plastic hoses, in particular infusion hoses, with a casing (2, 3) in which a pressure chamber (1) is provided, in which there is a membrane (5) and which is supplied with gas via an electrically activated valve (25) in a supply pipe, and with a forward- and backward-moving ram (12) having on the one hand a flat head (6) resting on the membrane (5) and, on the other hand, a peripheral grip (18) which acts upon a hose passage (17) under spring action, so that, with the membrane (5) under gas pressure and/or under the spring action of the hose passage (17) it is released or blocked, characterized in that, when the blocking device is used for an air trap, the pressure springs (9a, 9b) acting on the rams (12) have a circuit current passing through them, whereby in case of interruption, at least one pressure spring (9a, 9b) interrupts the current to it, thus blocking the hose passage (17).

2. Blocking device for an air trap in accordance with Claim 1, characterized in that the casing (2, 3) has to casing parts (2, 3) connected with each other and having each a recess, whereby a plate-shaped membrane (5) is clamped by them, forming, on the one hand, the pressure chamber (1) and, on the other hand, a recess (4) accommodating the flat head (6) and the pressure springs (9a, 9b).

3. Blocking device for an air trap in accordance with Claim 1 or 2, characterized in that two concentrically internal pressure springs (9a, 9b) act on the flat head (6).

4. Blocking device for an air trap in accordance with Claim 3, characterized in that pressure springs (9a, 9b) are isolated from each other.

5. Blocking device for an air trap in accordance with one of Claims 1-4, characterized in that the ram (12) reaches through a pod (14) arranged on a casing lid (13); the pod (14) serves as a passage for a hose (16).

6. Blocking device for an air trap in accordance with Claim 5, characterized in that the pod (14), with bilateral inward vaulting (15) for the hose (16) to pass through, has a transverse fin (19) in the middle and perpendicular to it; the transverse fin (19) acts together with the peripheral grip (18) as the hose clamp.

7. Blocking device for an air trap in accordance with one of the previous claims, characterized in that on either side of the hose (16) there is a photoelectric barrier which is open when coloured liquid passes through the hose (16) and is closed, via gas inlet control, when air bubbles pass.

8. Blocking device for an air trap in accordance with one of the previous claims, characterized in that at the passage of the hose (16) there are mountings on either side for inserting and removing the hose.

9. Blocking device for an air trap in accordance with one of the previous claims, characterized in that a reflective barrier (33) and a time measuring and comparison control is provided in the recess (4), whereby in case of a deviation between a desired value and an actual value, venting of the pressure chamber (1) is effected in order to activate the flat head (6) next to the membrane (5).

## Revendications

1. Dispositif d'arrêt pour tuyau en matière plastique, en particulier pour tuyau d'injection, comportant un boîtier (2,3), dans lequel est prévue une chambre de compression (1), dans laquelle est disposée une membrane (5) approvisionnée en gaz par une vanne (25) actionnée électriquement et disposée dans une conduite d'alimentation, comportant une tige-poussoir (12) mobile selon un mouvement de va-et-vient qui d'un côté présente une tête plate (6) qui appuie sur la membrane (5), et de l'autre côté présente une griffe (18) qui, sous l'action d'un ressort, vient appuyer sur un passage de tuyau (17), de façon à ce que le passage de tuyau (17) soit libéré par la membrane (5) sous pression de gaz ou pincé sous l'action d'un ressort
caractérisé en ce que, lors de l'application du dispositif d'arrêt pour un piège à air, des ressorts de pression (9a,9b) agissant sur la tige-poussoir (12) sont parcourus respectivement par un courant de commande qui est coupé à la rupture d'au moins un ressort de pression (9a,9b), provoquant, par conséquent, la fermeture du passage de tuyau (17).

2. Dispositif d'arrêt pour un piège à air selon la revendication 1 caractérisé en ce que le boîtier (2,3) comprend deux parties de boîtier (2,3) liées entre elles présentant chacune un évidement, et entre lesquelles la membrane (5) en forme d'assiette est encastrée constituant d'un côté la chambre de compression (1) et de l'autre côté un évidement (4) accueillant la tête plate (6) et les ressorts de pression (9a,9b).

3. Dispositif d'arrêt pour un piège à air selon les revendications 1 ou 2, caractérisé en ce que deux ressorts de pression (9a,9b) disposés l'un dans l'autre de façon concentrique agissent sur la tête plate (6).

4. Dispositif d'arrêt pour un piège à air selon la revendication 3 caractérise en ce que les ressorts de pression (9a,9b) sont isolés l'un de l'autre.

5. Dispositif d'arrêt pour un piège à air selon l'une quelconque des revendications 1 à 4 caractérise en ce que la tige-poussoir (12) s'étend à travers une douille (14) disposée sur un couvercle de boîtier (13), laquelle sert de passage à un tuyau (16).

6. Dispositif d'arrêt pour un piège à air selon la revendication 5 caractérisé en ce que la douille (14) présente des encoches bilatérales (15) pour le passage du tuyau (16) et, centrée perpendiculairement à celles-ci, une traverse (19), laquelle provoque en combinaison avec la griffe (18) la fermeture du tuyau (16).

7. Dispositif d'arrêt pour un piège à air selon l'une quelconque des revendications précédentes caractérisé en ce qu'il dispose d'un barrage photoélectrique sur le tuyau (16), qui en présence de liquide coloré parcourant le tuyau (16) est ouvert et au passage de bulles d'air se ferme et commande, en plus, l'arrêt de l'admission de gaz.

8. Dispositif d'arrêt pour un piège à air selon l'une quelconque des revendications précédentes caractérisé en ce que des attaches sont prévues des deux côtés du passage du tuyau (16) pour son insertion et son enlèvement.

9. Dispositif d'arrêt pour un piège à air selon l'une quelconque des revendications précédentes caractérisé en ce que l'évidement (4) comporte un barrage à réflexion (33) ainsi qu'une commande de mesure et d'asservissement qui en cas de divergence entre une valeur instantanée et une valeur de consigne provoque une mise à l'atmosphère de la chambre de compression (1), pour actionner la tête plate (6) accolée à la membrane (5).
